# EUROPEAN PATENT SPECIFICATION

(11) **EP 1 843 754 B1**
(45) Date of publication and mention of the grant of the patent: **03.08.2011**
(21) Application number: 06700941.5
(22) Date of filing: 24.01.2006
(51) Int. Cl.: A61K 9/20, A61K 45/06, A61P 9/12

(54) **NEW PHARMACEUTICAL COMPOSITION CONTAINING CANDESARTAN CILEXETIL AS LIPOPHILIC CRYSTALLINE SUBSTANCE**
NEUE PHARMAZEUTISCHE ZUSAMMENSETZUNG MIT CANDESARTAN-CILEXETIL ALS LIPOPHILE KRISTALLINE SUBSTANZ
NOUVELLE COMPOSITION PHARMACEUTIQUE CONTENANT CANDESARTAN CILEXETIL EN TANT QUE SUBSTANCE CRISTALLINE LIPOPHILE

(30) Priority: 26.01.2005 SI 200500021
(43) Date of publication of application: 17.10.2007
(73) Proprietor: LEK Pharmaceuticals d.d., 1526 Ljubljana (SI)
(72) Inventor: JERALA-STRUKELJ, Zdenka, 4211 Mavcice (SI); LEGEN, Igor, 1290 Grosuplje (SI)
(74) Representative: Oser, Andreas
(86) International application number: PCT/EP2006/000587
(87) International publication number: WO 2006/079496

(56) References cited:
- EP-A- 0 546 358
- WO-A-00/06126
- WO-A-97/37688
- US-A1- 2001 010 825
- US-A1- 2004 131 675

## Description

### FIELD OF THE INVENTION

The present invention from the field of pharmaceutical industry relates to the pharmaceutical composition comprising pharmaceutically active lipophylic substances, which are susceptible to degradation while being formulated, that is during the process of incorporating aforesaid substance into a composition, particularly into a finished dosage form, preferably into tablets. In particular the invention relates to the pharmaceutical compositions comprising active lipophylic substance, which is candesartan cilexetil, and optionally another active pharmaceutical ingredient, and to processes for their preparation. Specifically the invention relates to the use of small amounts of carrageenan in the process for manufacturing of aforesaid pharmaceutical compositions.

### BACKGROUND OF THE INVENTION

Candesartan cilexetil is an example of a lipophylic substance used as an antihypertensive agent and its therapeutic uses were disclosed in US 5,196,444, which also disclosed a crystalline form of candesartan cilexetil. It is believed that crystals of candesartan cilexetil are deformed by the elevated pressure during the tableting, which causes degradation /decomposition of the active substance during processing and/or storage and/or at the elevated temperature, which may be manifested as lowering the content thereof or increase of amount of impurities or related substances. US 5,534,534 describes incorporation of an oily substance having a lower melting point into a formulation containing candesartan cilexetil. The melting point of said oily substance selected from hydrocarbons, higher fatty acids, higher alcohols, fatty acid esters of polyhydric alcohols, higher alcohol ethers of polyhydric alcohols and polymers or copolymers of alkylene oxide ranges from 20 to 90 ° C. Not wishing to be bound by the theory it is believed that the oily substance probably melts during the tableting as a result of the raised temperature caused by the friction between the particles and that this molten substance probably attenuates the friction between the crystals of candesartan cilexetil and/or between the crystals of candesartan cilexetil and other particles in the tableting mixture and consequently minimizes crystalline disorders of candesartan cilexetil crystals. The same approach could be used in manufacturing a pharmaceutical composition of antihypertensive agent in combination with diuretic such as manidipine hydrochloride or hydrochlorothiazide.

### DISCLOSURE OF THE INVENTION

Present invention discloses new pharmaceutical compositions comprising candesartan cilexetil, and optionally another active pharmaceutical ingredient such as a diuretic and processes for their preparation.

The present invention provides a solid pharmaceutical composition according to claim 1. Further it provides a pharmaceutical composition in a form of a tablet according to claim 5. Even further it provides use of a composition according to claim 8 and of a carrageenan according to claim 9. Also, the present invention provides a process for preparing a stable pharmaceutical composition by tableting according to claim 10. The preferred embodiments are set forth in the sub-claims.

The solid pharmaceutical composition comprises from 2 to 20 % of a carrageenan.

Carrageenan is used in preferred amount around 10% to 1000% relative in weight to active, preferably in comparable amount, while the active pharmaceutical ingredient is candesartan cilexetil, and another optional active pharmaceutical ingredient is preferably hydrochlorotiazide or manidipine hydrochloride.

Specifically a solid pharmaceutical composition comprises candesartan cilexetil, preferably in amount of up to 20% by weight and up to 20 % by weight of a carrageenan and from 40 to 80% by weight of one or more diluents and from 2 to 25% by weight of one or more disintegrants and up to 5% by weight of one or more binders.

Most specifically the solid pharmaceutical composition comprises candesartan cilexetil and optionally or hydrochlorothiazide and up to 20 %, preferably up to 10%, more preferably between 2% and 5% by weight of a carrageenan and from 40 to 80% by weight of one or more diluents and from 2 to 25% by weight of one or more disintegrants and up to 5% by weight of one or more binders, preferably where it comprises of from 1 to 20% by weight of candesartan cilexetil, from 1 to 20% by weight of carrageenan, from 40 to 80% by weight of one or more diluents, from 2% to 25% by weight of one or more disintegrants and from 0,5% to 5% by weight of one or more binders.

The composition would comprise from 1 to 20% by weight of candesartan cilexetil, optionally from 1% to 20% by weight of hydrochlorotiazide and from 1 to 20% by weight of carrageenan.

Composition described above may additionally comprise together from 40 to 80% by weight of lactose and starch, from 2% to 25% by weight of sodium carboxymethylcellulose and from 0,5% to 5% by weight of povidone.

Preferably the pharmaceutical composition will be in a form of a tablet, comprising between about 1% and about 10% by weight of candesartan cilexetil and between 2% and 20% of carrageenan. More preferably it will additionally to the active ingredient and carrageenan comprise one or more inactive ingredients selected from group consisting of lactose, starch, povidone, carboxymethylcellulose sodium and magnesium stearate.

Specific steps in the process described above are:
a) providing a granulate comprising candesartan cilexetil;
b) mixing said granulate with one or more components where one of the components is carrageenan;
c) tableting the mixture obtained in previous step.

The pharmaceutical composition comprising candesartan cilexetil and carrageenan according to the invention is suitable for use in treating hypertension.

### DETAILED DESCRIPTION OF THE INVENTION

Stable formulations of an active pharmaceutical ingredient, such as described above have been developed by preparing pharmaceutical compositions comprising among excipients hydrophilic substances, preferably substances with hydrocolloidal properties which are conveniently selected from natural or synthetic polysaccharides with suitable properties, such as ability of swelling in an aqueous solvents which stabilize aforementioned active pharmaceutical ingredients against degradation or decomposition during the manufacturing of a pharmaceutical composition and/or during storage and/or at elevated temperatures. Prefered example of such stabilizing excipient is carrageenan, which is preferably added to the granulate in relatively small amounts before manufacturing of a finished solid dosage form. In a specific example carrageenan present in a pharmaceutical composition comprising candesartan cilexetil allows the application of low pressures during the tableting process without affecting the quality of the tablets. The pressures are selected to be low enough to prevent the crystalline disorders of candesartan cilexetil particles, such as below 20 kN, preferably below 10 kN, in certain embodiments preferably below 7 kN.

Carrageenans possess hydrophilic properties (which means that they interact with water in a manner that they for example freely mix with it and/or swell in it and/or form a gel, absorpt it) and hydrocolloidal properties (meaning forming colloidal dispersion, and or forming colloidal gel when dispered in water) and preferably have m.p. above 90 ° C (e.g. no significant endothermic changes are observed on DSC thermogram for carrageenan upon heating to approximately 100 ° C). Carrageenans are natural polysaccharides extracted from algae. They consist of the sulfate esters of galactose and 3,6-anhydrogalactose copolymers, linked alpha-1,3 and beta-1,4 in the polymer. They may be dried and ground to required specifications. Typical carrageenans are for example sold under tradenames Gelcarin1 GP-379 NF, Gelcarin GP-812 NF, and Gelcarin GP-911 NF, Viscarin1 GP-109 NF, and Viscarin GP-209 NF by FMC Corp.

Solid pharmaceutical composition in accordance with our invention comprise active pharmaceutical ingredient in amount up to 80% , preferably up to 25% more preferably from 1 to 10% by weight optionally in combination with another active pharmaceutical ingredients in a pharmaceutically acceptable carrier which comprises a relatively low amount of up to 20%, preferably from 2 to 20%, preferably less than 10%, most preferably about 5% of carrageenan. The pharmaceutical carrier can suitably comprise other inactive ingredients, preferably from 10 to 80%, more preferably from 40 to 60% of one or more diluents or fillers, such as lactose monohydrate, from 2 to 25%, more preferably above 10% of one or more disintegrants, such as starch or carboxylmethylcellulose sodium, up to 5%, preferably up to 2,5% of one or more suitable binders, such as povidone, one or more glidants, pigments and any other commonly used excipients.

Optional other inactive ingredients (excipients) may function as different fillers, binders, disintegrants, glidants, lubricants and/or excipients that enhance the absorption of drugs from gastrointestinal tract. Fillers may be selected from microcrystalline cellulose, powdered cellulose, lactose, starch, pregelatinized starch, sucrose, glucose, mannitol, sorbitol, calcium phosphate, calcium hydrogen phosphate, aluminium silicate, sodium chloride, potassium chloride, calcium carbonate, calcium sulphate, dextrates, dextrin, maltodextrin, glycerol palmitostearate, hydrogenated vegetable oil, kaolin, magnesium carbonate, magnesium oxide, polymethacrylates, talc, and others. Preferred fillers are starch and lactose monhydrate. Suitable binders may be starch, pregelatinized starch, gelatine, sodium carboxymethylcellulose, polyvinylpyrrolidone, alginic acid, sodium alginate, acacia, carbomer, dextrin, ehylcellulose, guar gum, hydrogenated vegetable oil, methylcellulose, hydroxyethylcellulose, hydroxypropylcellulose, hydroxypropylmethylcellulose, glucose syrup, magnesium aluminium silicate, maltodextrin, polymethacrylates. Preferably starch and polyvinylpyrrolidone are used. Suitable disintegrants may be selected from starch, pregelatinized starch, sodium starch glycolate, sodium carboxymethylcellulose, cross-linked sodium carboxymrethylcellulose, calcium carboxymethylcellulose, methylcellulose, microcrystalline cellulose, powdered cellulose, polacrilin potassium, cross-linked polivinylpyrrolidone, alginic acid, sodium alginate, colloidal silicon dioxide, guar gum, magnesium aluminium silicate, and others. Preferred disintegrant is sodium carboxymethylcellulose. Suitable glidants may be magnesium stearate, calcium stearate, aluminium stearate, stearic acid, palmitic acid, cetanol, stearol, polyethylene glycols of different molecular weights, magnesium trisilicate, calcium phosphate, colloidal silicon dioxide, talc, powdered cellulose, starch and others.. Suitable lubricants may be selected from stearic acid, calcium, magnesium, zinc or aluminium stearate, siliconized talc, glycerol monostearate, glycerol palmitostearate, hydrogenated castor oil, hydrogenated vegetable oil, mineral oil, light mineral oil, polyethylene glycol, sodium benzoate, sodium lauryl sulphate, sodium stearyl fumarate, talc and others. Preferred lubricants is magnesium stearate. Suitable absorption enhancers may be selected from surface active agents, fatty acids, middle chain glycerides, steroide detergents (salts of bile salts), acyl carnitine and alcanoloil choline (esters of carnitine and choline and fatty acids with middle chain and long chain). N-acyl derivatrives of alpha-amino acids and N-acyl derivatives of non-alpha-amino acids, chitosanes and other mucoadhesive polymers.

This invention is in specific embodiment an orally administrable tablet comprising following amounts of candesartan cilexetil: 2, 4, 8.16 or 32 mg.

The pharmaceutical compositions of our invention can be prepared as follows: In a granulator equipped with stirrer a powder mixture of one or more excipients such as diluents, disintegrants, binders is charged together with an active pharmaceutical ingredient. The mixture is granulated with a granulating liquid and to the granulating mixture or to the granules a stabilizing substance carrageenan is added optionally together with any other commonly used excipeints. Finaly the tablets are made by a gentle tableting process, that is the one where the main pressure is at maximum 20 kN, preferably 10 kN.

In specific embodiment of the invention a mixture of lactose with candesartan cilexetil, corn starch, povidone and pigment is granulated with water and dried in a fluid bad to give granules. To dried granules are added carrageenan, carboxymethylcellulose sodium and magnesium stearate. Tableting of thus obtained mixture is performed on a rotary tableting machine with 10 mm diameter flat-faced punches using the main pressure maximum 10 kN.

More specifically the present invention relates in an embodiment to a combination of two active ingredients, for example candesartan cilexetil as a angiotensin II antagonist with hydrochlorothiazide as a diuretic to achieve especially remarkable synergistic effects. The tablets are prepared as described above where the second active ingredient, alone or mixed with some other excipients is suitably added to the mixture to form granulate or to the already formed granulate. Hydrochlorothiazide presents 3-10 w.w.% in the tablets.

Within the scope of invention are also the alternatives, such as direct compression of powders or preparation of pellets and their incorporation into a finished dosage form.

The present invention is exemplified in the following examples of pharmaceutical compositions comprising candesartan cilexetil:

### Example 1: Candesartan cilexetil 32 mg

| **Dose** | **32 mg** | **%** |
|---|---|---|
| Candesartan cilexetil | 32.00 mg | 10.0 |
| Lactose monohydrate | 217.00 mg | 67.8 |
| Corn starch | 40.00 mg | 12.5 |
| Ferric oxide yellow | 0.40 mg | 0.1 |
| Povidone | 9.60 mg | 3.0 |
| Carrageenan | 12.00 mg | 3.8 |
| Carboxylmethylcellulose sodium | 8.00 mg | 2.5 |
| Magnesium stearate | 1.00 mg | 0.3 |
| Total | 320.00 mg | 100.0 |

### Example 2: Candesartan cilexetil 16 mg

| **Dose** | **16 mg** | **%** |
|---|---|---|
| Candesartan cilexetil | 16.00 mg | 10.0 |
| Lactose monohydrate | 108.50 mg | 67.8 |
| Corn starch | 20.00 mg | 12.5 |
| Ferric oxide yellow | 0.20 mg | 0.1 |
| Povidone | 4.80 mg | 3.0 |
| Carrageenan | 6.00 mg | 3.8 |
| Carboxylmethylcellulose sodium | 4.00 mg | 2.5 |
| Magnesium stearate | 0.50 mg | 0.3 |
| Total | 160.00 mg | 100.0 |

### Example 3: Candesartan cilexetil 8 mg

| **Dose** | **8 mg** | **%** |
|---|---|---|
| Candesartan cilexetil | 8.00 mg | 5.0 |
| Lactose monohydrate | 116,60 mg | 72.9 |
| Corn starch | 20.00 mg | 12.5 |
| Ferric oxide yellow | 0.10 mg | 0.1 |
| Povidone | 4.80 mg | 3.0 |
| Carrageenan | 6.00 mg | 3.7 |
| Carboxylmethylcellulose sodium | 4.00 mg | 2.5 |
| Magnesium stearate | 0.50 mg | 0.3 |
| Total | 160.00 mg | 100.0 |

### Example 4: Candesartan cilexetil 4 mg

| **Dose** | **4 mg** | **%** |
|---|---|---|
| Candesartan cilexetil | 4.00 mg | 2.5 |
| Lactose monohydrate | 120.70 mg | 75.5 |
| Corn starch | 20.00 mg | 12.5 |
| Povidone | 4.80 mg | 3.0 |
| Carrageenan | 6.00 mg | 3.7 |
| Carboxylmethylcellulose sodium | 4.00 mg | 2.5 |
| Magnesium stearate | 0.50 mg | 0.3 |
| Total | 160.00 mg | 100.0 |

### Example 5: Candesartan cilexetil 2 mg

| **Dose** | **2 mg** | **%** |
|---|---|---|
| Candesartan cilexetil | 2.00 mg | 1.2 |
| Lactose monohydrate | 122.70 mg | 76.8 |
| Corn starch | 20.00 mg | 12.5 |
| Povidone | 4.80 mg | 3.0 |
| Carrageenan | 6.00 mg | 3.7 |
| Carboxylmethylcellulose sodium | 4.00 mg | 2.5 |
| Magnesium stearate | 0.50 mg | 0.3 |
| Total | 160.00 mg | 100.0 |

### Example 6: Candesartan cilexetil 32 mg

| **Dose** | **32 mg** | **%** |
|---|---|---|
| Candesartan cilexetil | 32.00 mg | 8.0 |
| Lactose monohydrate | 279.25 mg | 69.8 |
| Corn starch | 50.00 mg | 12.5 |
| Ferric oxide yellow | 0.50 mg | 0.1 |
| Povidone | 12.00 mg | 3.0 |
| Carrageenan | 15.00 mg | 3.8 |
| Carboxylmethylcellulose sodium | 10.00 mg | 2.5 |
| Magnesium stearate | 1.25 mg | 0.3 |
| Total | 400.00 mg | 100.0 |

Tablets comprising besides candesartan cilexetil also another active ingredient, for example a diuretic such as manodipine or hydrochlorotiazide can be prepared as described in the following examples:

### Example 7: Candesartan 8 mg/ Hydrochlorothiazide 12.5 mg

| **Dose** | **8 mg** |
|---|---|
| Candesartan cilexetil | 8.00 mg |
| Hydrochlorothiazide | 12.50 mg |
| Lactose monohydrate | 104.10mg |
| Corn starch | 20.00 mg |
| Ferric oxide yellow | 0.10 mg |
| Povidone | 4.80 mg |
| Carrageenan | 6.00 mg |
| Carboxylmethylcellulose sodium | 4.00 mg |
| Magnesium stearate | 0.50 mg |
| Total | 160.00 mg |

### Example 8: Candesartan 16 mg/ Hydrochlorothiazide 12.5 mg

| **Dose** | **16 mg** |
|---|---|
| Candesartan cilexetil | 16.00 mg |
| Hydrochlorothiazide | 12.50 mg |
| Lactose monohydrate | 96.10 mg |
| Corn starch | 20.00 mg |
| Ferric oxide yellow | 0.10 mg |
| Povidone | 4.80 mg |
| Carrageenan | 6.00 mg |
| Carboxylmethylcellulose sodium | 4.00 mg |
| Magnesium stearate | 0.50 mg |
| Total | 160.00 mg |

Carrageenan has proven satisfactorily as demonstarted by following stability data: during the storage at 60° C for 14 days the tablet prepared without carrageenan (Comparative example) contain 5.34% of degradation products related to candesartan cilexetil, while the tablet prepared with carrageenan (Example 1) contain only 1,63% of degradation products related to candesartan cilexetil.

### Comparative example: Candesartan cilexetil 32 mg without carragenaan

| **Materials** | **Per tablet** |
|---|---|
| Candesartan cilexetil | 32.00 mg |
| Lactose monohydrate | 196.80 mg |
| Sodium lauryl sulfate | 3.20 mg |
| Microcristalline cellulose | 64.00 mg |
| Povidone | 9.60 mg |
| Aerosil | 0.8 mg |
| Sodium starch glycolate | 12.60 mg |
| Mg-stearate | 1.00 mg |
| Total | 320.00 mg |

## Claims

1. The solid pharmaceutical composition comprising candesartan cilexetil and optionally another active pharmaceutical ingredient and up to 20 % by weight of a carrageenan and from 40 to 80% by weight of one or more diluents and from 2 to 25% by weight of one or more disintegrants and up to 5% by weight of one or more binders.

2. The solid pharmaceutical composition according to claim 1, **characterized in that** the it comprises of from 1 to 20% by weight of candesartan cilexetil, from 1 to 20% by weight of carrageenan, from 40 to 80% by weight of one or more diluents, from 2% to 25% by weight of one or more disintegrants and from 0.5% to 5% by weight of one or more binders.

3. The solid pharmaceutical composition according to any of the previous two claims **characterized in that** the it comprises from 1 to 20% by weight of candesartan cilexetil, optionally from 1% to 20% by weight of hydrochlorotiazide and from 1 to 20% by weight of carrageenan.

4. The solid pharmaceutical composition according to previous claims **characterized in that** it additionally comprises together from 40 to 80% by weight of lactose and starch, from 2% to 25% by weight of sodium carboxymethylcellulose and from 0.5% to 5% by weight of povidone.

5. A pharmaceutical composition in a form of a tablet, comprising between about 1% and about 10% by weight of candesartan cilexetil and between 2% and 20% of carrageenan.

6. The pharmaceutical composition according to previous claim, **characterized in that** amount of carrageenan is about 4%.

7. The pharmaceutical composition according to previous claim, **characterized in that** it additionally comprises one or more inactive ingredients selected from group consisting of lactose, starch, povidone, carboxymethylcellulose sodium and magnesium stearate..

8. Use of a composition according to any of the previous claims for the manufacturing of a medicament.

9. Use of carrageenan in the manufacturing of a stable pharmaceutical composition comprising candesartan cilexetil.

10. Process for preparing a stable pharmaceutical composition by tableting, by first providing a granulate comprising candesartan cilexetil, **characterized in that** the carrageenan is added to the granulate before tableting and **in that** it is made by the tableting process where main pressure is at maximum 20 kN.

11. The process according to claim 10, **characterized in that** it contains following steps:
a) providing a granulate comprising candesartan cilexetil;
b) mixing said granulate with one or more components where one of the components is carrageenan;
c) tableting the mixture obtained in previous step.

## Patentansprüche

1. Feste pharmazeutische Zusammensetzung, die Candesartan-Cilexetil und wahlweise einen anderen wirksamen pharmazeutischen Bestandteil, sowie bis zu 20 Gew.-% eines Carrageens und 40 bis 80 Gew.-% eines oder mehrerer Streckmittel und 2 bis 25 Gew.-% eines oder mehrerer Zerfallsbeschleuniger und bis zu 5 Gew.-% eines oder mehrerer Bindemittel umfasst.

2. Feste pharmazeutische Zusammensetzung gemäß Anspruch 1, **dadurch gekennzeichnet, dass** sie 1 bis 20 Gew.-% Candesartan-Cilexetil, 1 bis 20 Gew.-% Carrageen, 40 bis 80 Gew.-% eines oder mehrerer Streckmittel, 2 bis 25 Gew.-% einen oder mehrerer Zerfallsbeschleuniger und 0,5 bis 5 Gew.-% eines oder mehrerer Bindemittel umfasst.

3. Feste pharmazeutische Zusammensetzung gemäß irgendeinem der vorangehenden zwei Ansprüche, **dadurch gekennzeichnet, dass** sie 1 bis 20 Gew.-% Candesartan-Cilexetil, wahlweise 1 bis 20 Gew.-% Hydrochlorthiazid, sowie 1 bis 20 Gew.-% Carrageen umfasst.

4. Feste pharmazeutische Zusammensetzung gemäß den vorangehenden Ansprüchen, **dadurch gekennzeichnet, dass** sie zusätzlich zugleich 40 bis 80 Gew.-% Lactose und Stärke, 2 bis 25 Gew.-% Natriumcarboxymethylcellulose und 0,5 bis 5 Gew.-% Povidon umfasst.

5. Pharmazeutische Zusammensetzung in Form einer Tablette, die zwischen etwa 1 und etwa 10 Gew.-% Candesartan-Cilexetil und zwischen 2 und 20 Gew.-% Carrageen umfasst.

6. Pharmazeutische Zusammensetzung gemäß dem vorangehenden Anspruch, **dadurch gekennzeichnet, dass** die Menge an Carrageen etwa 4% beträgt.

7. Pharmazeutische Zusammensetzung gemäß dem vorangehenden Anspruch, **dadurch gekennzeichnet, dass** sie zusätzlich einen oder mehrere nicht-wirksame Bestandteile umfasst, die aus der aus Lactose, Stärke, Povidon, Natriumcarboxymethylcellulose und Magnesiumstearat bestehenden Gruppe ausgewählt sind.

8. Verwendung einer Zusammensetzung gemäß irgendeinem der vorangehenden Ansprüche für die Herstellung eines Medikaments.

9. Verwendung von Carrageen bei der Herstellung einer stabilen pharmazeutischen Zusammensetzung, die Candesartan-Cilexetil umfasst.

10. Verfahren zum Herstellen einer stabilen pharmazeutischen Zusammensetzung durch Tablettieren, indem zuerst ein Candesartan-Cilexetil umfassendes Granulat bereitgestellt wird, **dadurch gekennzeichnet, dass** das Carrageen vor dem Tablettieren zu dem Granulat zugegeben wird, und dass sie durch ein Tablettierungsverfahren, in dem der Hauptdruck maximal 20 kN beträgt, hergestellt wird.

11. Verfahren gemäß Anspruch 10, **dadurch gekennzeichnet, dass** es folgende Schritte beinhaltet:
a) Bereitstellen eines Granulats, das Candesartan-Cilexetil umfasst;
b) Mischen des Granulats mit einem oder mehreren Bestandteil(en), wobei einer der Bestandteile Carrageen ist;
c) Tablettieren der im vorangehenden Schritt erhaltenen Mischung.

## Revendications

1. La composition pharmaceutique solide comprenant du candésartan cilexétil et de façon optionnelle un autre ingrédient pharmaceutique actif et jusqu'à 20 % en poids d'un carraghénane et entre 40 et 80 % en poids d'un ou plus de diluants et entre 2 et 25 % en poids d'un ou plus de délitants et jusqu'à 5 % en poids d'un ou plus de liants.

2. La composition pharmaceutique solide selon la revendication 1, **caractérisée en ce que** ladite composition comprend entre 1 et 20 % en poids de candésartan cilexétil et entre 1 et 20 % en poids de carraghénane , entre 40 et 80 % en poids d'un ou plus de diluants, entre 2 et 25 % en poids d'un ou plus de délitants et entre 0,5 % et 5 % en poids d'un ou plus de liants.

3. La composition pharmaceutique solide selon n'importe laquelle des deux revendications précédentes, **caractérisée en ce que** ladite composition comprend entre 1 et 20 % en poids de candésartan cilexétil , de façon optionnelle entre 1 % et 20 % en poids de hydrochlorothiazide et entre 1 et 20 % en poids de carraghénane.

4. La composition pharmaceutique solide selon les revendications précédentes **caractérisée en ce que** ladite composition comprend en outre ensemble entre 40 et 80 % en poids de lactose et d'amidon, entre 2 et 20 % en poids de cellulose de carboxyméthyle de sodium et entre 0,5 % et 5 % en poids de povidone.

5. Une composition pharmaceutique sous la forme d'un comprimé, comprenant entre environ 1 % et environ 10 % en poids de candésartan cilexétil et entre 2 % et 20 % de carraghénane .

6. La composition pharmaceutique selon la revendication précédente, **caractérisée en ce que** la quantité de carraghénane s'élève à environ 4 %.

7. La composition pharmaceutique selon la revendication précédente, **caractérisée en ce que** ladite composition comprend un ou plus d'ingrédients sélectionnés du groupe composé de lactose, amidon, povidone, cellulose de carboxyméthyle de sodium et de stéarate de magnésium.

8. Utilisation d'une composition selon n'importe laquelle des revendication précédentes pour fabriquer un médicament.

9. Utilisation de carraghénane dans la fabrication d'une composition pharmaceutique stable comprenant le candésartan cilexétil.

10. Procédé de fabrication d'une composition pharmaceutique stable en fabriquant des comprimés en fournissant en premier un granulé comprenant du candésartan cilexétil , **caractérisé en ce que** le carraghénane est ajouté au granulé avant la fabrication du comprimé et **en ce que** ladite composition est fabriquée par un procédé de fabrication de comprimés où la pression principale s'élève au maximum à 20 kN.

11. Le procédé selon la revendication 9, **caractérisé en ce que** ledit procédé est composé des étapes suivantes :
a)mise à disposition de candésartan cilexétil ;
b)mélanger ledit granulé avec un ou plus de composants où un des composants est carraghénane ;
c)Transformation du mélange, obtenu par les étapes précédentes, en comprimés.
